(19) **Europäisches Patentamt / European Patent Office / Office européen des brevets**

(11) **EP 4 685 483 A1**

(12) **EUROPEAN PATENT APPLICATION**
published in accordance with Art. 153(4) EPC

(43) Date of publication:
**28.01.2026 Bulletin 2026/05**

(21) Application number: **24880889.1**

(22) Date of filing: **02.02.2024**

(51) International Patent Classification (IPC):
***G01N 33/48*** (2006.01)       ***G16B 15/30*** (2019.01)

(86) International application number:
**PCT/CN2024/075478**

(87) International publication number:
**WO 2025/086502 (01.05.2025 Gazette 2025/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.10.2023   CN 202311394888**

(71) Applicants:
- **China-Japan Friendship Hospital (China-Japan Friendship Institute of Clinical Medicine)**
  **Beijing 100029 (CN)**
- **Institute of Materia Medica, Chinese Academy of Medical Sciences and Peking Union Medical College**
  **Beijing 100050 (CN)**

(72) Inventors:
- **LIU, Lihong**
  **Beijing 100029 (CN)**

- **ZHANG, Jinlan**
  **Beijing 100029 (CN)**
- **SHENG, Ning**
  **Beijing 100029 (CN)**
- **LIU, Xiao**
  **Beijing 100029 (CN)**
- **MA, Ziying**
  **Beijing 100029 (CN)**
- **SU, Nan**
  **Beijing 100029 (CN)**
- **YANG, Ting**
  **Beijing 100029 (CN)**
- **WANG, Xiaoxue**
  **Beijing 100029 (CN)**
- **WANG, Chen**
  **Beijing 100029 (CN)**

(74) Representative: **Lapienis, Juozas**
**MSP Europe UAB**
**21-92 Seimyniskiu Str.**
**09236 Vilnius (LT)**

(54) **SPHINGOLIPID METABOLIC MARKER, ANALYSIS METHOD THEREFOR AND USE THEREOF**

(57)    A sphingolipid metabolism biomarker, an analytical method for this biomarker and its application are provided, which can be effectively used for early diagnosis, disease progression monitoring, and prognostic evaluation of asthma, with high sensitivity, rapidity, convenience, and accurate and reliable results. The sphingolipid metabolism biomarker includes at least one of the following substances: galactosyl ceramide d18:1/16:0, galactosyl ceramide d18:1/14:0.

**EP 4 685 483 A1**

FIG. 1

**Description**

**CROSS REFERENCE TO THE RELATED APPLICATIONS**

This application is the national phase entry of International Application No. PCT/CN2024/075478, filed on February 2, 2024, which is based upon and claims priority to Chinese Patent Application No. 202311394888.7, filed on October 26, 2023, the entire contents of which are incorporated herein by reference.

**TECHNICAL FIELD**

The invention relates to the technical field of biomedical detection technology, particularly to a sphingolipid metabolic biomarker, an analytical method for this biomarker, and its application in the dynamic monitoring of asthma progression.

**BACKGROUND**

Bronchial asthma (asthma) is a common chronic respiratory disease that seriously endangers human health, characterized by airway inflammation and hyperreactivity. In recent years, its incidence rate has been continuously rising worldwide, affecting more than 300 million people globally, with over 300,000 deaths from asthma each year. There are approximately 50 million asthma patients in China, and the mortality rate of asthma is much higher than in developed countries. Early diagnosis, disease monitoring, and standardized treatment of asthma play an important role in improving the control level of asthma and enhancing the quality of life of patients.

Currently, the primary diagnostic methods for asthma include blood cell analysis, pulmonary function monitoring, allergen testing, and clinical symptoms such as wheezing, shortness of breath, chest tightness, or coughing. However, the diagnosis of pathological staging and grading relies solely on clinical manifestations without biochemical indicators. It is worth noting that these methods are only measures taken after the onset of asthma and cannot predict disease risk, resulting in significant lag time. Therefore, a novel, non-invasive dynamic monitoring biomarker that can easily detect, diagnose, stage, grade, and predict prognosis is of crucial importance for early risk warning, disease course management, and prognostic evaluation of asthma.

Metabolomics can sensitively and comprehensively measure the changes in endogenous metabolites during an organism's physiological and pathological states. It allows for systematic investigation of the relationship between diseases and the composition of metabolites in the body, facilitating a comprehensive understanding and evaluation of disease states and the discovery of new biomarkers. Therefore, metabolomics holds significant clinical value in disease diagnosis, risk prediction, and prognostic assessment.

**SUMMARY**

In order to overcome the defects of the prior art, the technical problem to be solved by the present invention is to provide a sphingolipid metabolism biomarker, which can be effectively used for early diagnosis, disease progression monitoring, and prognostic evaluation of asthma, with high sensitivity, rapidity, convenience, and accurate and reliable results.

The technical scheme of the invention is as follows.

A sphingolipid metabolism biomarker includes at least one of the following substances: galactosyl ceramide d18:1/16:0, galactosyl ceramide d18:1/14:0.

An analytical method for the sphingolipid metabolic biomarker, wherein a fitted regression curve obtained through logistic regression analysis:

$$Y = Logit(p)$$

$$= 37.604a - 1116.674b + 44.959c - 2.948d + 181.759e + 0.439f - 3.741$$

in which Y represents a diagnostic probability, Logit(p) represents a logistic regression function, a-f respectively represents the concentration ratio of sphingosine-1-phosphate to sphingolipid d18:1/24:0, the concentration ratio of phosphorylated ceramide sphingolipid d18:1/14:1 to sphingolipid d18:1/24:0, the concentration ratio of sphingolipid d18:1/20:0 to sphingolipid d18:1/24:0, the concentration ratio of sphingolipid d18:1/18:0 to sphingolipid d18:1/24:0, the concentration ratio of sphingolipid d18:1/16:0 to sphingolipid d18:1/24:0, the concentration ratio of sphingolipid d18:1/24:1 to sphingolipid d18:1/24:0.

In the invention the sphingolipid metabolic biomarker and analysis method can be effectively used for early diagnosis, disease progression monitoring, and prognostic evaluation of asthma. They exhibit high sensitivity, are rapid and convenient, and provide accurate and reliable results. The biomarker and method can be used for multiple monitoring during the clinical control period, chronic persistent period, and acute exacerbation period of asthma patients, providing a basis for clinical decision-making. At the same time, they lay a certain foundation for subsequent basic research and clinical research, thus possessing significant application and research value.

Its application is also provided which is applied in dynamic monitoring of the course of asthma.

Its application is also provided that a chip, test strip, or reagent kit used for dynamic monitoring of asthma progression obtains a ratio level of metabolites in a biological sample of a test subject and is used to assess the disease status of asthma in the test subject.

## BRIEF DESCRIPTION OF THE DRAWINGS

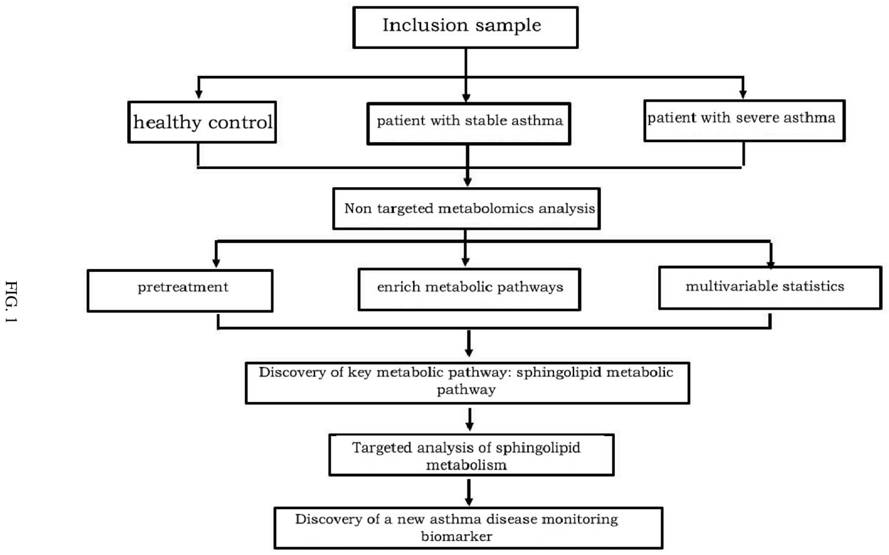

FIG. 1 shows a basic flowchart of the asthma metabolomics study in the invention.

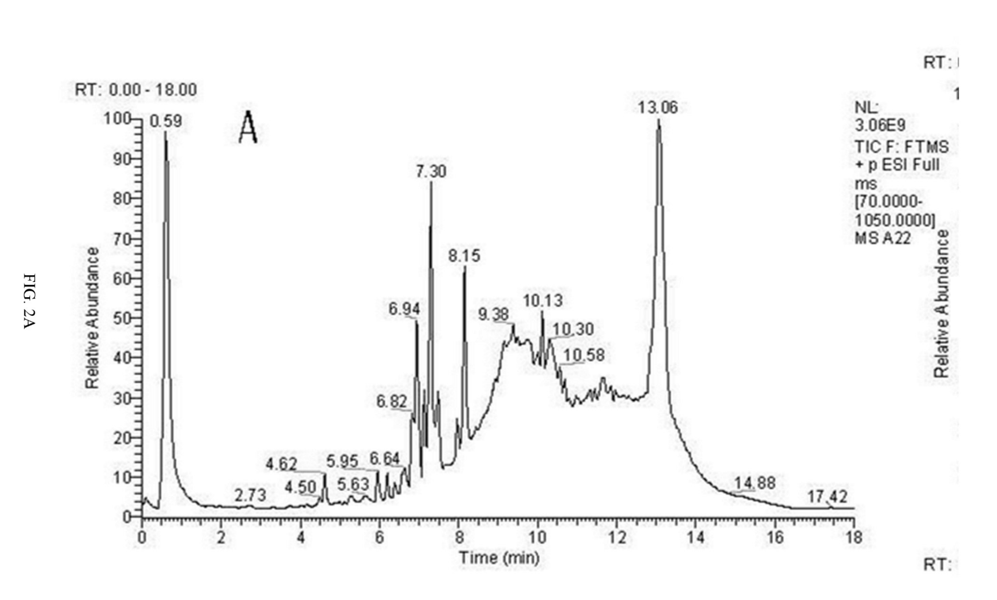

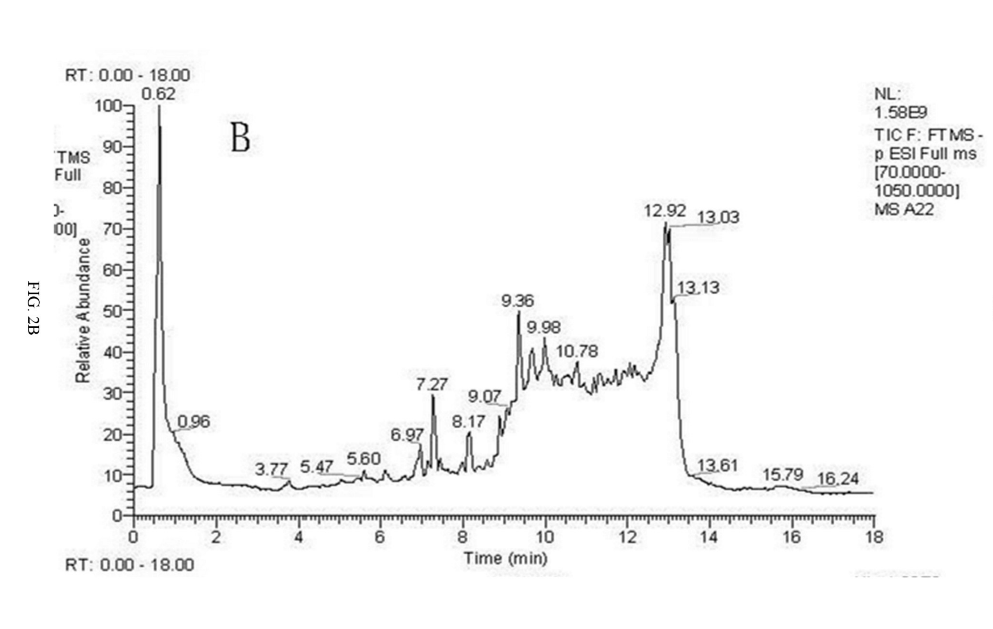

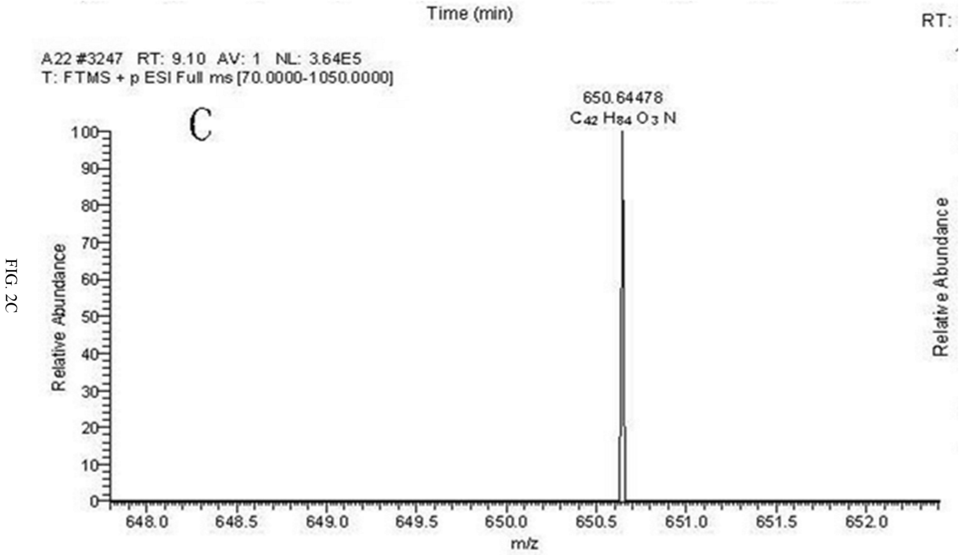

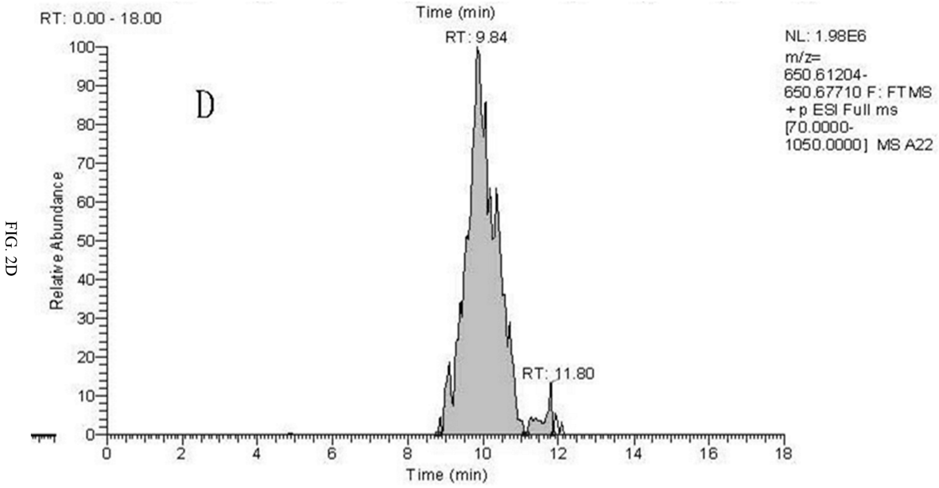

FIGS. 2A-2D show detection results of high-resolution mass spectrometry of a sample in an embodiment of the present invention. Specifically, FIG. 2A represents the total ion current of positive ions obtained from high-resolution mass spectrometry detection of the sample. FIG. 2B represents the total ion current of negative ions obtained from high-resolution mass spectrometry detection of the sample. FIG. 2C shows the corresponding precise mass number and molecular formula of sphingolipid (d18:1/24:0) obtained from high-resolution mass spectrometry detection of the sample. FIG. 2D presents the chromatogram of sphingolipid (d18:1/24:0) obtained from high-resolution mass spectrometry detection of the sample.

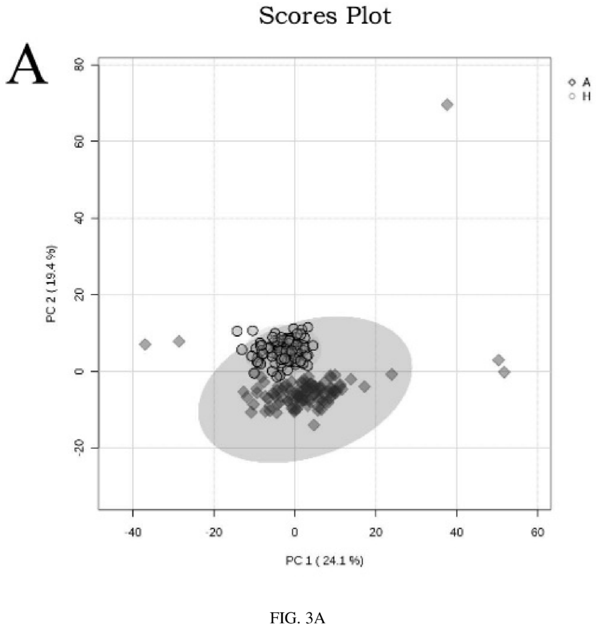

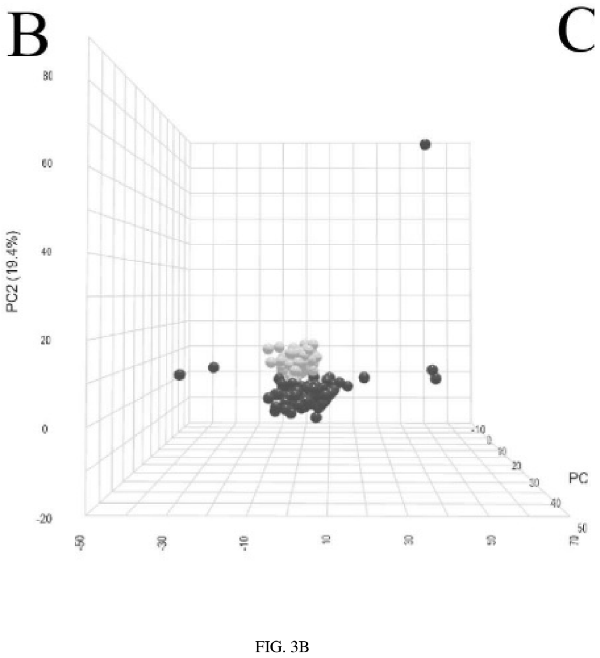

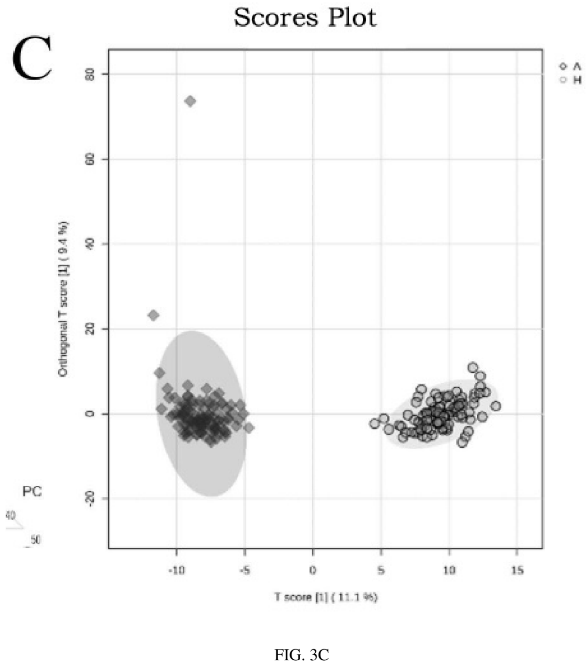

FIGS. 3A-3C show PCA and OPLS-DA model score plots for a healthy control group and an asthma group in an embodiment of the present invention. FIG. 3A is the PCA model score plot, where circles represent the healthy control group and diamonds represent the asthma group. FIG. 3B is a three-dimensional plot of the PCA, where light-colored spheres represent the healthy control group and dark-colored spheres represent the asthma group. From the above figures, it is evident that the model can clearly distinguish between healthy individuals and asthma patients. FIG. 3C is the OPLS-DA model score plot, where circles represent the healthy control group and diamonds represent the asthma group. The results show that there is a significant distinction between HC (healthy control) and asthma patients.

FIG. 4 shows the differential metabolic pathway diagram for asthma.

FIG. 5 shows the structural diagram of sphingolipid metabolites.

FIG. 6 shows the MRM extraction chromatogram of the target sphingolipid metabolites and the internal standard in a mixed human plasma sample.

FIG. 7 shows the standard curve of target sphingolipid metabolites based on the standard addition method.

FIG. 8 shows the regression curves fitted by multiple metabolic markers based on the sphingolipid ratio obtained through logistic regression analysis, as well as the results of ROC analysis.

FIG. 9 illustrates the changes in target sphingolipid metabolites in self-controlled patients with stable asthma (S) and severe asthma (A). Sphingolipid d18:1/20:0, sphingolipid d18:1/18:0, sphingolipid d18:1/16:0, sphingolipid d18:1/24:1 are all upregulated during the acute phase.

## DETAILED DESCRIPTION OF THE EMBODIMENTS

The sphingolipid metabolism biomarker includes at least one of the following substances: galactosyl ceramide d18:1/16:0, galactosyl ceramide d18:1/14:0.

Preferably, the sphingolipid metabolic marker also includes sphingosine-1-phosphate, phosphorylated ceramide sphingolipid d18:1/14:1, sphingolipid d18:1/20:0, sphingolipid d18:1/18:0, sphingolipid d18:1/16:0, sphingolipid d18:1/24:1, and their concentration ratios to sphingolipid d18:1/24:0, obtained through targeted metabolomics screening. Specifically, the sphingolipid metabolism biomarker also includes at least one of the following substances: sphingosine-1-phosphate obtained through targeted metabolomics screening, phosphorylated ceramide sphingolipid d18:1/14:1, sphingolipid d18:1/20:0, sphingolipid d18:1/18:0, sphingolipid d18:1/16:0, sphingolipid d18:1/24:1, the concentration ratio of sphingosine-1-phosphate to sphingolipid d18:1/24:0, the concentration ratio of phosphorylated ceramide sphingolipid d18:1/14:1 to sphingolipid d18:1/24:0, the concentration ratio of sphingolipid d18:1/20:0 to sphingolipid d18:1/24:0, the concentration ratio of sphingolipid d18:1/18:0 to sphingolipid d18:1/24:0, the concentration ratio of sphingolipid d18:1/16:0 to sphingolipid d18:1/24:0, and the concentration ratio of sphingolipid d18:1/24:1 to sphingolipid d18:1/24:0.

The analytical method for the sphingolipid metabolic biomarker, wherein a fitted regression curve obtained through logistic regression analysis:

$$Y = Logit(p)$$

$$= 37.604a - 1116.674b + 44.959c - 2.948d + 181.759e + 0.439f - 3.741$$

in which Y represents a diagnostic probability, Logit(p) represents a logistic regression function, a-f respectively represents the concentration ratio of sphingosine-1-phosphate to sphingolipid d18:1/24:0, the concentration ratio of phosphorylated ceramide sphingolipid d18:1/14:1 to sphingolipid d18:1/24:0, the concentration ratio of sphingolipid d18:1/20:0 to sphingolipid d18:1/24:0, the concentration ratio of sphingolipid d18:1/18:0 to sphingolipid d18:1/24:0, the concentration ratio of sphingolipid d18:1/16:0 to sphingolipid d18:1/24:0, the concentration ratio of sphingolipid d18:1/24:1 to sphingolipid d18:1/24:0.

In the invention the sphingolipid metabolic biomarker and analysis method can be effectively used for early diagnosis, disease progression monitoring, and prognostic evaluation of asthma. They exhibit high sensitivity, are rapid and convenient, and provide accurate and reliable results. The biomarker and method can be used for multiple monitoring during the clinical control period, chronic persistent period, and acute exacerbation period of asthma patients, providing a basis for clinical decision-making. At the same time, they lay a certain foundation for subsequent basic research and clinical research, thus possessing significant application and research value.

Its application is also provided which is applied in dynamic monitoring of the course of asthma.

Its application is also provided that a chip, test strip, or reagent kit used for dynamic monitoring of asthma progression obtains a ratio level of metabolites in a biological sample of a test subject and is used to assess the disease status of asthma in the test subject.

Preferably, the biological sample is plasma, serum, whole blood, dried whole blood spot, or dried plasma spot.

Preferably, the metabolite level in the biological sample of the test subject is obtained with one or more of the following methods: chromatography, spectroscopy, mass spectrometry, chemical analysis, and immunoassay.

Preferably, the chromatography method includes high-performance liquid chromatography, thin-layer chromatography, and gas chromatography; the spectroscopic method includes nuclear magnetic resonance spectroscopy, refractive index spectroscopy, ultraviolet spectroscopy, and near-infrared spectroscopy; the chemical analysis method includes electrochemical analysis and radiochemical analysis; the mass spectrometry method is triple quadrupole mass spectrometry, which involves first using a chromatographic column for gradient elution, and then collecting data under an electrospray ionization positive ion mode, in which positive ion scanning is used, and all metabolic biomarkers and internal standards are analyzed with a single injection for detection.

Preferably, a pretreatment method for the biological sample before detection includes: precisely aspirating 80 μL of plasma or serum, adding 10 μL of internal standard working solution, adding 480 μL of isopropanol/methanol solution 9:1, v/v, precipitating proteins, vortexing for 10 minutes, centrifuging at 12000 rpm for 10 minutes, collecting the supernatant, concentrating and evaporating to dryness under vacuum, reconstituting with 80 μL of methanol, vortexing for 10 minutes, centrifuging at 12000 rpm for 15 minutes, and obtaining the test solution for quantitative analysis.

Preferably, drawing 15-50 μL of human whole blood onto a dried blood spot collection card, and allowing it to dry at room temperature for 1-4 hours until the color turns deep red, thus obtaining a dried blood spot sample; using a punch to remove one or multiple dried blood spot samples with a diameter of 3-8 mm from the dried blood spot; adding the dried blood spot samples to a solid-phase extraction column with phospholipid/protein removal function, and eluting them with an organic reagent containing 5%-50% water.

The following provides a detailed description of the embodiments of the present invention.

**Embodiment 1**

Utilize non-targeted metabolomics technology to screen biomarkers and enrich metabolic pathways between asthmatic patients and healthy individuals.

1. Sample source:

After approval by the Ethics Committee of China-Japan Friendship Hospital, plasma samples are collected from 100 healthy controls (HC), 154 patients with stable asthma, and 112 patients with severe asthma. All participants are from China-Japan Friendship Hospital, and all asthma patients are diagnosed with bronchial asthma based on the "Guidelines for the Prevention and Treatment of Bronchial Asthma (2020 Edition)" after clinical assessment by physicians, excluding history of respiratory diseases such as tuberculosis, interstitial lung fibrosis, and chronic obstructive pulmonary disease. The age and gender of the HC are matched with those of the asthma patients to exclude metabolic differences caused by gender and age. Plasma collection is performed in the early morning in a fasting state. All samples are stored at -80°C for future use.

2. Main reagents:

LC/MS-grade acetonitrile is purchased from Merck Corporation, HPLC-grade methanol is purchased from Merck Corporation, and formic acid is purchased from CNW Corporation. Other reagents are all commercially available analytical reagents. Deionized water is prepared using the Milli-Q ultrapure water system from Millipore Corporation.

3. Research methods:

3.1 Sample preparation:

Sample pretreatment: 20 $\mu$L of plasma (from healthy control group, stable asthma group, and severe asthma group) is aspirated and added to 180 $\mu$L of precipitant containing an internal standard (methanol: acetonitrile = 1:1). The mixture is vortexed for 3 minutes and centrifuged at 13,000 rpm for 10 minutes. 100 $\mu$L of the supernatant is aspirated for non-targeted metabolomic analysis.

3.2 Chromatographic/Mass Spectrometric Conditions:

The detection is performed using a high-resolution mass spectrometer QE-Orbitrap. Chromatographic mobile phase: Mobile phase A is an aqueous solution containing 0.1% formic acid and 2.5 mmol/L ammonium formate, while mobile phase D is acetonitrile. The gradient elution program for sample determination is as follows: 0-1.0 min, 95% A; 1.0-5.0 min, 95%-40% A; 5.0-8.0 min, 40%-0% A; 8.0-11.0 min, 0% A; 11.0-14.0 min, 0%-40% A; 14.0-15.0 min, 40%-95% A; 15.0-18.0 min, 95% A. The analysis time is 0-18 min, with 5 $\mu$L of sample injected each time, and the flow rate is 0.25 mL/min.

Chromatographic column: ACQUITY BEH C18 1.7 $\mu$m, 2.1×50 mm, with a column temperature of 30°C. The temperature of the autosampler is maintained at 4°C. Data are collected in both positive and negative ion modes using an electrospray ion source (ESI), with a spray voltage of 3000 V; evaporation temperature of 350°C; capillary temperature of 350°C; S-lens RF of 50; resolution of the first-level full scan (Full scan) of 70000, and scanning range of 70-1050 m/z. Secondary data-dependent scanning (Full MS/dd-MS2) is performed with a resolution of 17500; AGC target of 1e5; Maximum TT of 50 ms; and NCE of 20, 40, 60. The ultra-high-resolution mass spectrometry detection results are shown in FIGS. 2A-2D.

3.3 Metabolic pathway analysis:

Using Metaboanalyst 5.0, the differences in endogenous metabolites in plasma samples are analyzed (from healthy control group, stable asthma group, and severe asthma group) to identify endogenous metabolites with a VIP value>1, P value<0.05, and Foldchange (FC)>1.5 or <0.5. Subsequently, the Pathway Analysis on the MetaboAnalyst 5.0 website is employed to explore the differential metabolic pathways in bone marrow tissue and bone marrow supernatant. Metabolic pathway with an Impact value greater than 0.1 are selected is a primary differential metabolic pathway associated with asthma.

4. Data processing and statistical analysis:

The identification of endogenous metabolites involves obtaining the precise mass number to five decimal places for each endogenous metabolite using high-resolution mass spectrometry mzCloud, identifying them based on their molecular formulas, and utilizing the database of over 1200 endogenous metabolites (including carbohydrates, lipids, fatty acids, amino acids, etc.) previously established by Tracefinder software. The peak areas of corresponding endogenous metabolites are automatically matched through the Tracefinder database, and then analyzed using the MetaboAnalyst5.0 website to draw an OPLS-DA model diagram, identifying metabolic pattern differences and obvious classification trends between healthy individuals and asthma patients. Finally, differential metabolic biomarkers are selected based on the criteria of VIP > 1, P < 0.05, FC > 1.5 or < 0.5.

5. Results:

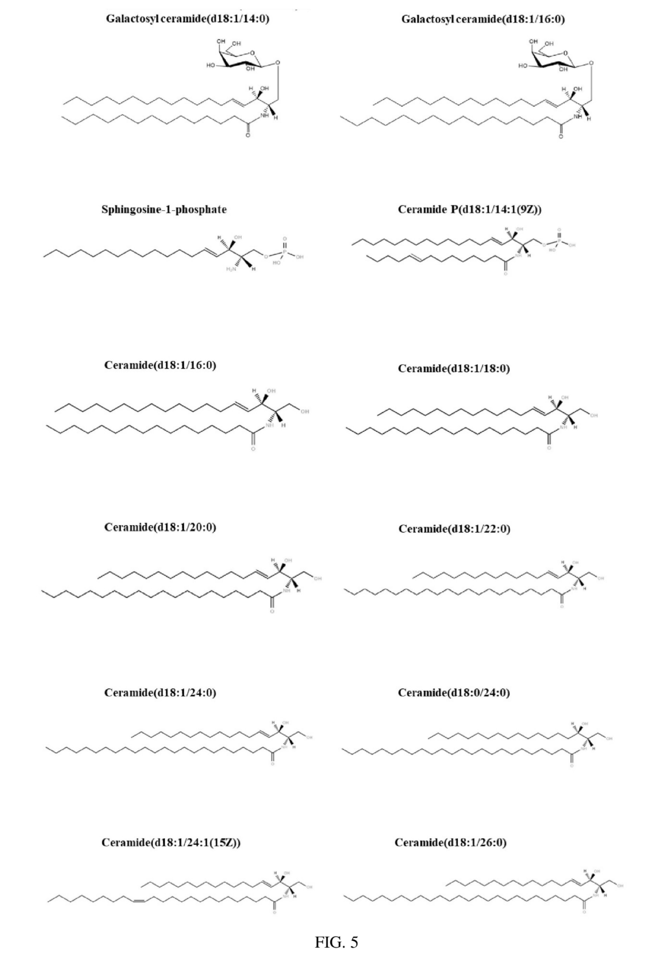

PCA is calculated using the MetaboAnalyst 5.0 website. The PCA results (FIG. 3A and FIG. 3B) show the differences between the plasma of the healthy control group and the asthma group. The OPLS-DA results (FIG. 3C) demonstrate a clear separation between the healthy control group and the asthma group. Endogenous substances with VIP > 1, P-value < 0.05, and FC > 1.5 or < 0.5 are selected for further analysis of the main differential metabolic pathways in asthma patients. Table 1 presents the change rates of differential metabolites in asthma obtained using the OPLS-DA model. The results show that there are a total of 29 differential metabolites. Compared to the healthy control group, the proportions of 10 endogenous substances in the asthma group mainly increase, while those of the other 19 substances mainly show a downward trend. The results indicate that the development of asthma lesions in the body affects the metabolic secretion of plasma endogenous substances, leading to significant changes in their contents, with the main altered components being lipid substances. Among them, the area under the ROC curve for galactosylceramide d18:1/16:0 and galactosylceramide d18:1/14:0 is as high as 0.97. The specific structures are shown in FIG. 5.

Tabel 1

| No. | Metabolite | VIP | P value | FC | ROC |
|---|---|---|---|---|---|
| 1 | galactosyl ceramide (d18:1/16:0) | 1.32 | 2.29E-15 | 2.48 | 0.97 |
| 2 | galactosyl ceramide (d18:1/14:0) | 1.4 | 1.80E-14 | 2.2 | 0.97 |
| 3 | PC(o-16:0/18:0) | 1.35 | 5.43E-16 | 2.0 | 0.96 |
| 4 | N-butyryl-L-homoserine lactone | 1.09 | 1.34E-40 | 1.81 | 0.96 |
| 5 | PC(20:3(5Z,8Z,11Z)/20:3(5Z,8Z,11Z)) | 1.4 | 1.55E-14 | 1.8 | 0.98 |
| 6 | sphingosine 1-phosphate | 1.14 | 5.68E-11 | 1.71 | 0.94 |
| 7 | SM(d18:0/20:2(11Z,14Z)) | 1.01 | 1.77E-06 | 1.7 | 0.80 |
| 8 | PC(o-22:0/20:4(8Z,11Z,14Z,17Z)) | 1.19 | 9.68E-15 | 1.67 | 0.95 |
| 9 | glycine | 1.07 | 1.65E-08 | 1.56 | 0.56 |
| 10 | SM(d18:0/18:0) | 1.37 | 7.15E-19 | 1.52 | 0.98 |
| 11 | Cis-5-tetradecyl carnitine | 1.52 | 3.66E-16 | 0.5 | 0.55 |
| 12 | Stearoyl ethanolamide | 1.77 | 5.27E-35 | 0.5 | 0.56 |
| 13 | SM (d18:0/16:1 (9Z) (OH)) | 1.74 | 7.22E-32 | 0.5 | 0.60 |
| 14 | PG(16:0/18:2(9Z,12Z)) | 1.34 | 3.42E-09 | 0.41 | 0.58 |
| 15 | PG(16:0/22:5(4Z,7Z,10Z,13Z,16Z)) | 1.71 | 3.67E-19 | 0.40 | 0.58 |
| 16 | Sphingolipid (d18:1/24:0) | 1.40 | 2.0E-42 | 0.28 | 0.78 |
| 17 | LysoPE(0:0/22:2(13Z,16Z))/LysoPE(22:2(1 3Z,16Z)/0:0) | 1.53 | 1.92E-17 | 0.27 | 0.72 |
| 18 | Homocitrulline | 1.24 | 9.18E-23 | 0.21 | 0.77 |
| 19 | Oleoyl glycine | 1.84 | 5.25E-32 | 0.18 | 0.86 |
| 20 | 2-Methoxyestradiol | 1.25 | 9.75E-15 | 0.17 | 0.64 |
| 21 | PG (18:0/20:3 (8Z, 11Z, 14Z)) | 1.6 | 2.61E-37 | 0.16 | 0.93 |
| 22 | dopa | 1.58 | 7.73E-22 | 0.15 | 0.84 |
| 23 | pantothenic acid | 1.96 | 2.85E-29 | 0.13 | 0.83 |
| 24 | N - (3-oxohexanoyl) homoserine lactone | 1.93 | 3.80E-37 | 0.12 | 0.61 |
| 25 | 3-hydroxy-3-methyl-2-oxobutyric acid | 2.0 | 1.14E-42 | 0.12 | 0.80 |
| 26 | 1-Deoxyglucose | 2.04 | 1.50E-34 | 0.11 | 0.92 |

(continued)

| No. | Metabolite | VIP | P value | FC | ROC |
|-----|-----------|-----|---------|-----|-----|
| 27 | 2,4-diaminobutyric acid | 1.76 | 6.75E-22 | 0.08 | 0.87 |
| 38 | Cytosine | 2.04 | 2.2E-42 | 0.07 | 0.80 |
| 29 | Acryloyl carnitine | 2.31 | 9.47E-44 | 0.06 | 0.98 |

Study on the Differences in Endogenous Substance Metabolic Pathways:

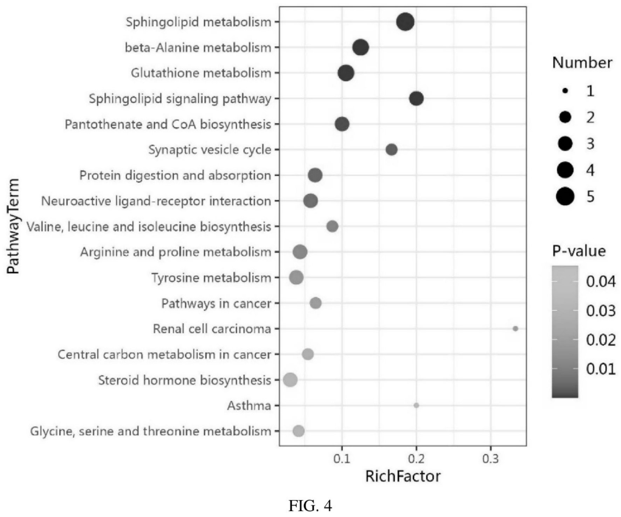

FIG. 4 shows the analysis of metabolic pathways in asthma using MetaboAnalyst5.0. The results in Table 3 show that there are significant differences in metabolic pathways between the healthy control group and the asthma group with an Impact value greater than 0.1, mainly consisting of 5 pathways, indicating that the two groups mainly affect the metabolism and synthesis of sphingolipids. The analysis results show that asthma affects the metabolism and synthesis of sphingolipids, leading to changes in endogenous substance content in the patient's body. Therefore, metabolites on the sphingolipid metabolism pathway are ultimately selected as targets for targeted metabolomics analysis to distinguish healthy individuals from asthma patients.

Tabel 2

| No. | Metabolic pathway | Total | Hits | FDR | Impact |
|-----|-------------------|-------|------|-----|--------|
| 1 | sphingolipid metabolism | 21 | 5 | 0.000832 | 0.32252 |
| 2 | Tyrosine metabolism | 42 | 3 | 0.42346 | 0.20015 |
| 3 | Glycerophospholipid metabolism | 36 | 2 | 0.98449 | 0.19895 |
| 4 | Arginine and proline metabolism | 38 | 3 | 0.40509 | 0.14269 |
| 5 | glutathione metabolism | 28 | 4 | 0.029707 | 0.10311 |

**Embodiment 2**

Validation of biomarkers between asthma patients and healthy individuals using targeted sphingolipid metabolomics analysis.

(1). Sample source:

Collect plasma samples from 100 healthy controls, 98 stable asthma patients, and 94 severe asthma patients. All participants are from the China-Japan Friendship Hospital.
The asthma patients were diagnosed with bronchial asthma according to the Guidelines for the Prevention and Treatment of Bronchial Asthma (2020 Edition), and the history of respiratory diseases such as pulmonary tuberculosis, pulmonary interstitial fibrosis and chronic obstructive pulmonary disease is excluded after evaluation by clinicians.
Match the age and gender of HC with asthma patients to exclude metabolic differences caused by gender and age. The blood collection time is in the early morning fasting state.
Collect fasting plasma samples from the subjects and store them in a -80°C freezer for future use.

(2). Sample testing:

1. Instrument information:

Mass spectrum name: IVD triple quadrupole liquid mass spectrometry system for clinical medicine (K6460, Serial No.: SG1638K003, Agilent Corporation) is equipped with electric spray ion source (ESI), and liquid phase name: 1200 RRLC system ultra high performance liquid chromatograph. ORTEX-2GENE Vortex Mixer (Scientific Industry Corporation); METLER TOLEDO AG135 electronic balance (Mettler AG Corporation, Switzerland); Milli-Q Ultra Pure Water Device (No. 041572, Merck Corporation).
2. Sample pretreatment: Precisely aspirate 80 $\mu$L of plasma or serum, add 10 $\mu$L of internal standard working solution, add 480 $\mu$L of isopropanol/methanol solution (9:1, v/v) to precipitate protein, vortex for 10 minutes, centrifuge at 12000 rpm for 10 minutes, take the supernatant, vacuum concentrate and evaporate, dissolve in 80 $\mu$L of methanol, vortex for 10 minutes, centrifuge at 12000 rpm for 15 minutes, and obtain the test solution for quantitative analysis. Dried blood tablets: Take 15-50 μL of human whole blood droplets onto a dried blood spot collection card, dry at room temperature for 1-4 hours until the color turns dark red, and obtain a dried blood spot sample. Use a puncher to remove one or more dried blood spot samples with a diameter of 3-8 mm from the dried blood spot. Add the dried blood spot sample to a solid-phase extraction column with phospholipid/protein removal function, and use an organic reagent containing 5% -50% water for elution.

3. Instrument parameters:

Chromatographic conditions: The column size is Agilent Poroshell 300 SB-C8 (2.1 × 75 mm 5μm), the column temperature is 40°C, and the temperature of the automatic sampler is maintained at 4°C. The mobile phase A is water containing 0.1% formic acid and 1.0 mM ammonium acetate, and the mobile phase D is methanol. The elution time is 0-2.0 min, with a 40% gradient elution to 70% D, 2.0-8.0 min, 70% gradient elution to 90% D, 8.0-10.0 min, and 90% gradient elution to 96% D. Each injection is 10 μL, and the flow rate is 0.4 mL/min.

Mass spectrum conditions: spray ion source (ESI) positive ion detection mode, MRM mode, during data collection, spray voltage is 2500-5000 V, dryer temperature is 150-350°C, sheath gas temperature is 250-400°C, spray gas flow rate is 10-50 psi, sheath gas flow rate is 5-20 L/min, capillary temperature is 150-320°C, MRM ion pair monitors target sphingolipid metabolites.

(3). Result analysis:

1. Standard curves and linear ranges of metabolites of various esters

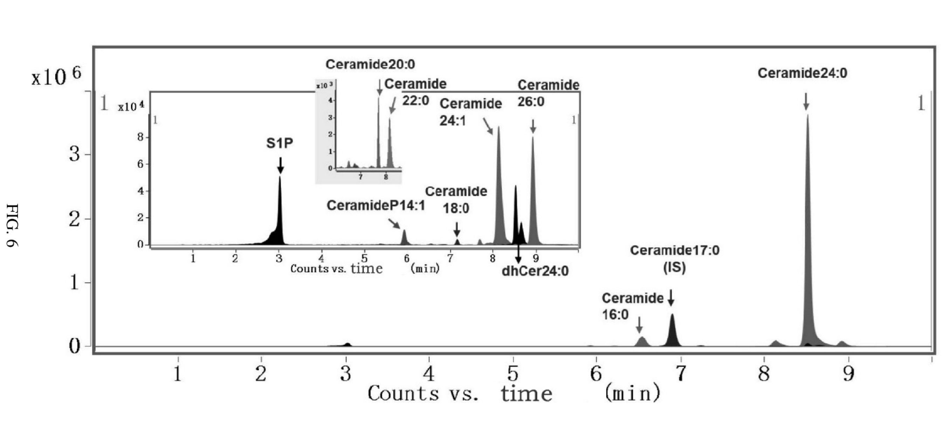

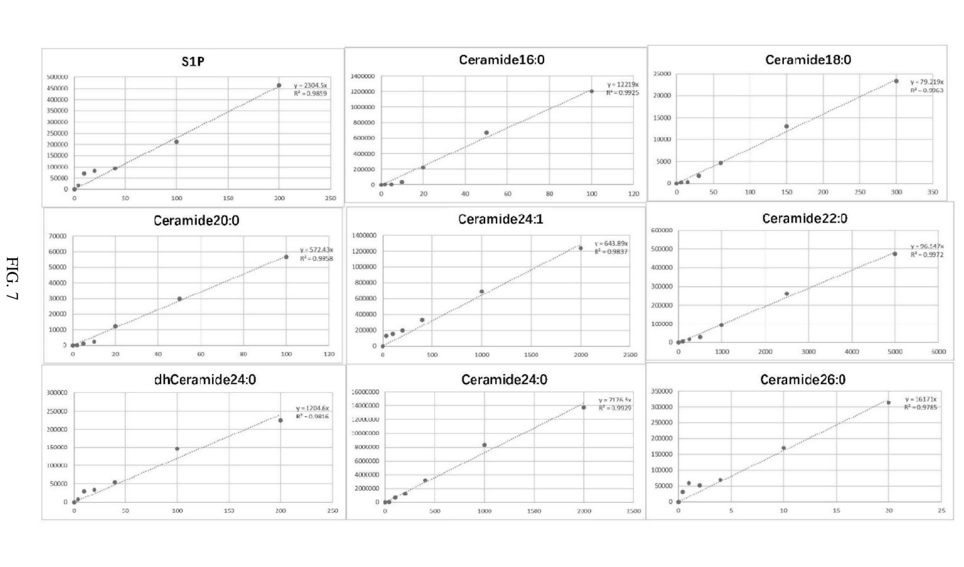

The structure diagram of sphingolipid metabolites determined by targeted metabolomics is shown in FIG. 5, and the extraction diagram of target sphingolipid metabolites and internal standard MRM in mixed human plasma samples is shown in FIG. 6. Matrix standards of various concentrations of sphingolipid metabolites are added to the mixed plasma samples, and the samples are continuously injected from low to high concentrations. The standard curve is calculated using the standard addition method, with the concentration of each analyte added as the horizontal axis (X) and the peak area of the analyte subtracted from the background peak area as the vertical axis (Y). The linear equation and linear correlation coefficient $R2$ of the matrix standard curve are obtained (FIG. 7). The results show that sphingosine 1-phosphate (S1P), sphingolipids d18:1/16:0 (Ceramics 16:0), d18:1/18:0 (Ceramics 18:0), d18:1/20:0 (Ceramics 20:0), d18:1/22:0 (Ceramics 22:0), d18:1/24:0 (Ceramics 24:0), d18:0/24:0 (dhCeramics 24:0), d18:1/24:1 (Ceramics 24:1), and d18:1/16:0 (Ceramics 26:0) are within their respective concentration ranges, and their peak areas are linearly correlated with concentration. The coefficient $R2$ is greater than 0.98 (Table 3).

Table 3

| Target analyte | Add concentration range (ng/mL) | Standard Curve | $R^2$ |
|---|---|---|---|
| S1P | 4-200 | y = 2304.5x | 0.9859 |
| Ceramide 16: 0 | 2-100 | y = 12219x | 0.9925 |
| Ceramide18:0 | 5-300 | y = 79.219x | 0.9963 |
| Ceramide20:0 | 2-100 | y = 572.43x | 0.9958 |
| Ceramide22:0 | 100-5000 | y = 96.547x | 0.9972 |
| Ceramide24:0 | 40-2000 | y = 7176.5x | 0.9929 |
| Ceramide24:1 | 40-2000 | y = 643.89x | 0.9837 |
| Ceramide26:0 | 0.4-20 | y = 16171x | 0.9850 |
| dhCeramide24:0 | 4-200 | y = 1204.6x | 0.9816 |

2. Quality control sample analysis

Using mixed plasma samples as quality control samples: the within batch precision RSD of each measured sphingolipids is less than 12.45%; The inter batch precision RSD is less than 13.25%, which meets the quality control requirements for endogenous metabolite analysis (Table 4).

Table 4

| batch | Sample | S1P | Ceramide P14:1 | Ceramide 16:0 | Ceramide 18:0 | Ceramide 20:0 | Ceramide 24:1 | Ceramide 22:0 | dhCeramide24:0 | Ceramide 24:0 | Ceramide 26:0 |
|---|---|---|---|---|---|---|---|---|---|---|---|
| | | Area | Area | Area | Area | Area | Area | Area | Area | Area | Area |
| 1 | QC01 | 382336 | 65505 | 934445 | 17800 | 19817 | 664694 | 22752 | 175240 | 16232265 | 506562 |
| | QC02 | 353891 | 68358 | 981203 | 18326 | 17915 | 663514 | 19620 | 180852 | 16060146 | 501144 |
| | QC03 | 333463 | 71196 | 1013396 | 20039 | 18822 | 638738 | 19076 | 184969 | 15866494 | 493066 |
| | RSD (%) | 6.9 | 4.2 | 4.1 | 6.3 | 5.1 | 2.2 | 9.7 | 2.7 | 1.1 | 1.4 |
| 2 | QC04 | 352776 | 72806 | 1007303 | 19625 | 17518 | 651992 | 20829 | 194214 | 16129298 | 483391 |
| | QC05 | 311519 | 76003 | 1027359 | 19874 | 20091 | 643097 | 21331 | 207004 | 16281636 | 488686 |
| | QC06 | 334719 | 74973 | 1027687 | 21305 | 19503 | 628577 | 18937 | 200107 | 16234319 | 474393 |
| | RSD (%) | 6.2 | 2.2 | 1.1 | 4.5 | 7.1 | 1.8 | 6.2 | 3.2 | 0.48 | 1.5 |
| 3 | QC07 | 459554 | 61442 | 868056 | 19386 | 21636 | 640900 | 17536 | 208109 | 15834765 | 506369 |
| | QC08 | 451962 | 58596 | 841575 | 19376 | 18222 | 596731 | 15885 | 187625 | 15562341 | 503141 |
| | QC09 | 449939 | 57542 | 774388 | 16564 | 19646 | 564517 | 18293 | 171829 | 14361576 | 484066 |
| | QC10 | 411868 | 58504 | 775643 | 15861 | 16056 | 542523 | 15284 | 177031 | 14971699 | 492596 |
| | RSD (%) | 4.8 | 2.9 | 5.8 | 10.4 | 12.5 | 7.3 | 8.4 | 8.6 | 4.3 | 2.1 |
| 4 | QC11 | 330319 | 63703 | 862348 | 15737 | 15234 | 538264 | 15073 | 167585 | 15055078 | 476626 |
| | QC12 | 394047 | 60024 | 827129 | 16181 | 17221 | 574693 | 18291 | 192371 | 15307676 | 491683 |
| | QC13 | 399628 | 57671 | 832865 | 17434 | 18335 | 566058 | 16195 | 181370 | 15425306 | 501861 |
| | RSD (%) | 10.3 | 5.0 | 2.3 | 5.4 | 9.3 | 3.4 | 9.9 | 6.9 | 1.2 | 2.6 |
| Between batches | RSD (%) | 13.3 | 10.5 | 10.6 | 9.9 | 9.4 | 7.6 | 12.9 | 6.9 | 3.8 | 2.2 |

3. Analysis of serum samples from asthma related individuals

Quantitative analysis is conducted on the target sphingolipid metabolites in plasma samples from healthy individuals (H), stable asthma patients (S), and severe asthma patients (A). The plasma concentrations of the target sphingolipid metabolites in each group of samples are determined and shown in Table 5. Further t-tests are conducted on target sphingolipid metabolites between groups, and the results show that there are significant differences in the t-tests of 1-phosphate sphingosine (S1P), phosphorylated ceramide sphingosine d18:1/14:1 (CeramideP4:1), sphingolipid d18:1/16:0 (Ceramide16:0), sphingolipid d18:1/18:0 (Ceramide20:0), sphingolipid d18:1/20:0 (Ceramide20:0), and sphingolipid d18:1/24:1 (Ceramide24:1) between severe asthma (A) and stable asthma (S) groups, as well as between severe asthma (A) and healthy individuals (H) groups ($p < 0.05$). 0.01) (Table 5). There is no significant difference in t-test of other sphingolipid metabolites ($p > 0.05$).

Further based on the ROC curve, diagnostic biomarkers for severe asthma are determined. SPSS software is used to set the group as the state variable, and the target concentration of sphingolipid metabolites is used as the test variable. A larger test result is used to indicate a more positive test for analysis. In large-scale data analysis, if the area under the ROC analysis curve is greater than 0.7, it can be considered that the indicator has good diagnostic ability. In the case of asymptotic significance $p < 0.05$ if the area under the ROC curve is greater than 0.65, it can also be considered that the indicator has good diagnostic ability. The ROC analysis area under the curve (AUC) of 1-phosphate sphingosine (S1P), phosphorylated ceramide sphingolipids d18:1/14:1 (Ceramide14:1), sphingolipids d18:1/16:0 (Ceramide16:0), sphingolipids d18:1/18:0 (Ceramide18:0), sphingolipids d18:1/20:0 (Ceramide20:0), and sphingolipids d18:1/24:1 (Ceramide24:1) between severe asthma phase (A) and stable asthma phase (S) is calculated (Table 6).

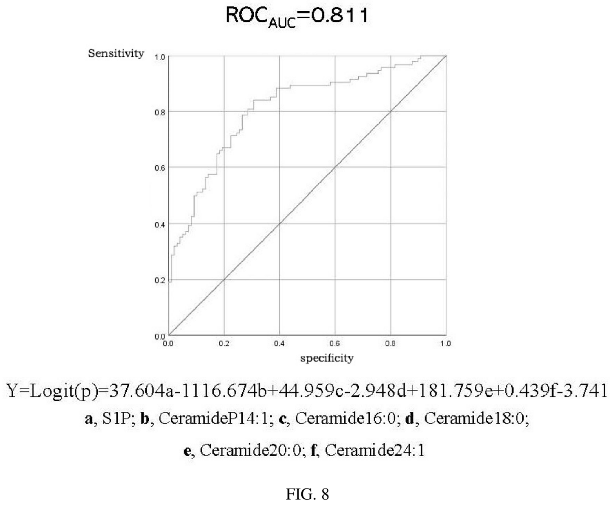

Based on the area under the ROC analysis curve, the diagnostic ability of sphingolipid biomarkers in severe asthma is evaluated, and further comparisons are made. (1) ROC analysis is performed using biomarker concentrations directly measured in the sample. (2) The ratio of biomarker concentration to sphingolipid d18:1/24:0 (Ceramide 24:0) concentration measured in the sample is subjected to ROC analysis. The summarized ROC analysis data are shown in Table 6. The results indicate that the ratio of biomarker concentration to sphingolipid 24:0 concentration can also be used as a biomarker for indicating severe asthma. Further logistic regression analysis is performed to obtain regression curves for multiple metabolic markers based on the ratio of sphingolipid markers to sphingolipid d18:1/24:0 concentration: $Y = Logit(p) = 37.604a - 1116.6748b + 44.959c - 2.948d + 181.759e + 0.439f - 3.741$ (Y represents a diagnostic probability, Logit(p) represents a logistic regression function, a-f respectively represents the concentration ratio of sphingosine-1-phosphate to sphingolipid d18:1/24:0, the concentration ratio of phosphorylated ceramide sphingolipid d18:1/14:1 to sphingolipid d18:1/24:0, the concentration ratio of sphingolipid d18:1/20:0 to sphingolipid d18:1/24:0, the concentration ratio of sphingolipid d18:1/18:0 to sphingolipid d18:1/24:0, the concentration ratio of sphingolipid d18:1/16:0 to sphingolipid d18:1/24:0, the concentration ratio of sphingolipid d18:1/24:1 to sphingolipid d18:1/24:0). The area under the ROC curve in the comparison between severe asthma stage (A) and stable asthma stage (S) after fitting is 0.811 (FIG. 8), which increases the accuracy of asthma disease diagnosis.

Table 5

| Group | Concentration | S1P ng/mL | CeramideP 14:1 ng/mL | Ceramide 16:0 ng/mL | Ceramide 18:0 ng/mL | Ceramide 20:0 ng/mL | Ceramide 24:1 ng/mL | Ceramide 22:0 ng/mL | dhCeramide24:0 ng/mL | Ceramide 24:0 ng/mL | Ceramide 26:0 ng/mL |
|---|---|---|---|---|---|---|---|---|---|---|---|
| Healthy people (H) | Average concentration | 145.7 | 5.1 | 81.3 | 212.9 | 26.7 | 892.8 | 240.6 | 157.5 | 2201.8 | 25.5 |
| Stable asthma phase (S) | Average concentration | 117.9 | 5.2 | 80.4 | 224.6 | 28.7 | 981.8 | 265.7 | 173.3 | 2376.7 | 27.7 |
| Severe asthma phase (A) | Average concentration | 127.7 | 5.8 | 98.2 | 379.8 | 48.3 | 1179.6 | 256.1 | 169.4 | 2310.7 | 26.2 |
| T-test | S/H | p<0.0 001 | ns | ns | ns | ns | p=0.043 | p=0.027 | p=0.0029 | p=0.0076 | ns |
| | A/H | p<0.0 001 | p=0.0002 | p<0.0001 | p<0.0001 | p<0.0001 | p<0.0001 | ns | p=0.0396 | ns | ns |
| | A/S | p=0.0 077 | p=0.002 | p<0.0001 | p<0.0001 | p<0.0001 | p=0.0002 | ns | ns | ns | ns |

Table 6

| Inter group comparison | Name | ROC analysis (direct comparison) | | ROC analysis (biomarker/C24 concentration ratio) | | Logistic regression (biomarker/C24 concentration ratio) |
|---|---|---|---|---|---|---|
| | | Region | Asymptotic significance | Region | Asymptotic significance | Region |
| Severe asthma phase (A) vs Stable asthma phase (S) | Ceramide 20:0 | 0.763 | <0.0001 | 0.795 | <0.0001 | 0.811 formula: Y=Logit(p) =37.604a-1116.674-b+ 44.959e-2.948-d+181.759e+ 0.439f-3.741 |
| | Ceramide 18:0 | 0.746 | <0.0001 | 0.752 | <0.0001 | |
| | Ceramide 16:0 | 0.709 | <0.0001 | 0.722 | <0.0001 | |
| | Ceramide 24:1 | 0.666 | <0.0001 | 0.706 | <0.0001 | |
| | S1P | 0.653 | <0.0001 | 0.686 | <0.0001 | |
| | Ceramide P14:1 | 0.656 | <0.0001 | 0.698 | <0.0001 | |

**Embodiment 3**

Specific case analysis

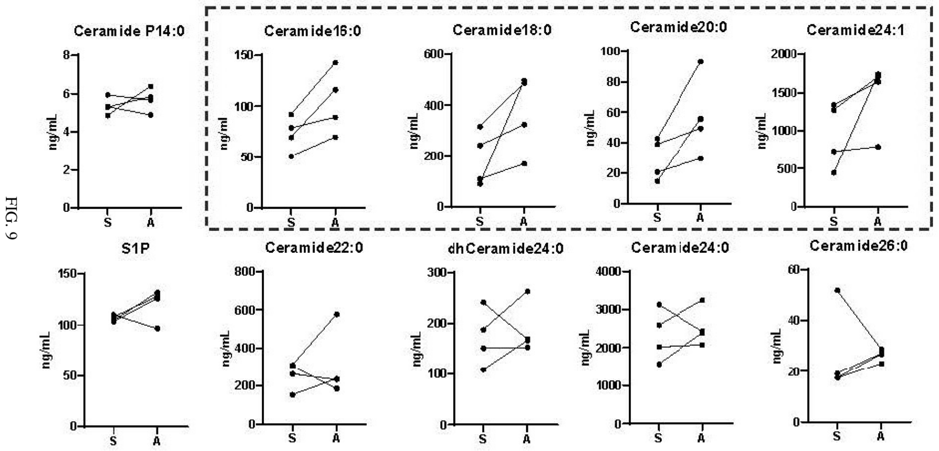

This experiment includes four self controlled patients, who are in different states of stable asthma stage (S) and severe asthma stage (A). The results show that the diagnostic biomarkers of sphingolipids d18:1/16:0, d18:1/18:0, d18:1/20:0, and d18:1/24:1 are upregulated in all four self controlled patients during severe asthma stage (A) (FIG. 9). Overall, the results indicate that sphingolipids d18:1/16:0, d18:1/18:0, d18:1/20:0, and d18:1/24:1 can become biomarkers for dynamic monitoring of asthma course.

**Embodiment 4**

In order to verify the detection effect of metabolic markers, the following grouping experiments were designed.
Using the same method as in Embodiment 2, 10 repeated tests are conducted on metabolic markers in the plasma of 15 suspected asthma patients. The metabolic markers are grouped as follows:

1) Single detection group of sphingolipids d18:1/20:0;

2) Single detection group for sphingolipids d18:1/18:0;

3) Single detection group of sphingolipids d18:1/16:0;

4) Single detection group of sphingolipids at 18:1/24:1;

5) Joint testing team.

Due to individual differences, as well as instrument and reagent errors during the detection process, there are often missed detections in the actual operation of metabolic marker detection, which can cause adverse consequences such as delays in the patient's condition. Based on this, in this example a statistical analysis of the missed detection rates of the five groups of metabolic markers mentioned above is conducted, and the specific results are shown in Table 7.

Table 7

| Grouping | Number of missed detections in 10 repeated tests | The missed detection rate |
|---|---|---|
| Single detection group of sphingolipids d18:1/20:0 | 3 | 30% |
| Single detection group for sphingolipids d18:1/18:0 | 4 | 40% |
| Single detection group of sphingolipids d18:1/16:0 | 5 | 50% |
| Single detection group of sphingolipids at 18:1/24:1 | 5 | 50% |

(continued)

| Grouping | Number of missed detections in 10 repeated tests | The missed detection rate |
|---|---|---|
| Joint testing team | 1 | 10% |

From Table 7, it can be seen that simultaneous detection of metabolic markers (sphingolipid composition) can effectively reduce the missed detection rate for asthma.

The above contents are only the preferable embodiments of the present invention, and do not limit the present invention in any manner. Any improvements, amendments and alternative changes made to the above embodiments according to the technical spirit of the present invention shall fall within the claimed scope of the present invention.

## Claims

1. A sphingolipid metabolic biomarker, comprising at least one of the following substances: galactosyl ceramide d18:1/16:0, galactosyl ceramide d18:1/14:0.

2. The sphingolipid metabolic biomarker according to claim 1, wherein the sphingolipid metabolic marker also comprises sphingosine-1-phosphate, phosphorylated ceramide sphingolipid d18:1/14:1, sphingolipid d18:1/20:0, sphingolipid d18:1/18:0, sphingolipid d18:1/16:0, sphingolipid d18:1/24:1, and their concentration ratios to sphingolipid d18:1/24:0, obtained through targeted metabolomics screening.

3. An analytical method for the sphingolipid metabolic biomarker according to claim 2, wherein a fitted regression curve obtained through logistic regression analysis:

$$Y = Logit(p) = 37.604a - 1116.674b + 44.959c - 2.948d + 181.759e + 0.439f - 3.741$$

in which Y represents a diagnostic probability, Logit(p) represents a logistic regression function, a-f respectively represents the concentration ratio of sphingosine-1-phosphate to sphingolipid d18:1/24:0, the concentration ratio of phosphorylated ceramide sphingolipid d18:1/14:1 to sphingolipid d18:1/24:0, the concentration ratio of sphingolipid d18:1/20:0 to sphingolipid d18:1/24:0, the concentration ratio of sphingolipid d18:1/18:0 to sphingolipid d18:1/24:0, the concentration ratio of sphingolipid d18:1/16:0 to sphingolipid d18:1/24:0, the concentration ratio of sphingolipid d18:1/24:1 to sphingolipid d18:1/24:0.

4. An application for the sphingolipid metabolic biomarker according to claim 1 or 2, wherein the sphingolipid metabolic markers are used for dynamic monitoring of the course of asthma.

5. An application for the sphingolipid metabolic biomarker according to claim 1 or 2, wherein a chip, test strip, or reagent kit used for dynamic monitoring of asthma progression obtains a ratio level of metabolites in a biological sample of a test subject and is used to assess the disease status of asthma in the test subject.

6. The application for the sphingolipid metabolic biomarker according to claim 5, wherein the biological sample is plasma, serum, whole blood, dried whole blood spot, or dried plasma spot.

7. The application for the sphingolipid metabolic biomarker according to claim 6, wherein the metabolite level in the biological sample of the test subject is obtained with one or more of the following methods: chromatography, spectroscopy, mass spectrometry, chemical analysis, and immunoassay.

8. The application for the sphingolipid metabolic biomarker according to claim 7, wherein the chromatography method comprises high-performance liquid chromatography, thin-layer chromatography, and gas chromatography;

the spectroscopic method comprises nuclear magnetic resonance spectroscopy, refractive index spectroscopy, ultraviolet spectroscopy, and near-infrared spectroscopy;
the chemical analysis method comprises electrochemical analysis and radiochemical analysis;
the mass spectrometry method is triple quadrupole mass spectrometry, which involves first using a chromatographic column for gradient elution, and then collecting data under an electrospray ionization positive ion mode, in which positive ion scanning is used, and all metabolic biomarkers and internal standards are analyzed with a single injection for detection.

9. The application for the sphingolipid metabolic biomarker according to claim 7, wherein a pretreatment method for the biological sample before detection comprises:

precisely aspirating 80 µL of plasma or serum, adding 10 µL of internal standard working solution,
adding 480 µL of isopropanol/methanol solution 9:1, v/v,
precipitating proteins, vortexing for 10 minutes,
centrifuging at 12000 rpm for 10 minutes, collecting the supernatant, concentrating and evaporating to dryness under vacuum,
reconstituting with 80 µL of methanol, vortexing for 10 minutes,
centrifuging at 12000 rpm for 15 minutes, and
obtaining the test solution for quantitative analysis.

10. The application for the sphingolipid metabolic biomarker according to claim 7, wherein drawing 15-50 µL of human whole blood onto a dried blood spot collection card, and allowing it to dry at room temperature for 1-4 hours until the color turns deep red, thus obtaining a dried blood spot sample;

using a punch to remove one or multiple dried blood spot samples with a diameter of 3-8 mm from the dried blood spot;
adding the dried blood spot samples to a solid-phase extraction column with phospholipid/protein removal function, and eluting them with an organic reagent containing 5%-50% water.

FIG. 1

FIG. 2A

FIG. 2B

EP 4 685 483 A1

FIG. 2C

19

FIG. 2D

EP 4 685 483 A1

Scores Plot

A

FIG. 3A

B

C

FIG. 3B

FIG. 3C

FIG. 4

Galactosyl ceramide(d18:1/14:0)

Galactosyl ceramide(d18:1/16:0)

Sphingosine-1-phosphate

Ceramide P(d18:1/14:1(9Z))

Ceramide(d18:1/16:0)

Ceramide(d18:1/18:0)

Ceramide(d18:1/20:0)

Ceramide(d18:1/22:0)

Ceramide(d18:1/24:0)

Ceramide(d18:0/24:0)

Ceramide(d18:1/24:1(15Z))

Ceramide(d18:1/26:0)

FIG. 5

FIG. 6

FIG. 7

$$Y=Logit(p)=37.604a-1116.674b+44.959c-2.948d+181.759e+0.439f-3.741$$

**a**, S1P; **b**, CeramideP14:1; **c**, Ceramide16:0; **d**, Ceramide18:0;

**e**, Ceramide20:0; **f**, Ceramide24:1

FIG. 8

FIG. 9

## INTERNATIONAL SEARCH REPORT

| International application No. |
| --- |
| **PCT/CN2024/075478** |

| A. | CLASSIFICATION OF SUBJECT MATTER |
| --- | --- |

G01N33/48(2006.01)i;  G16B15/30(2019.01)n

According to International Patent Classification (IPC) or to both national classification and IPC

| B. | FIELDS SEARCHED |
| --- | --- |

Minimum documentation searched (classification system followed by classification symbols)

IPC: G01N33/-、 G16B15/-

Documentation searched other than minimum documentation to the extent that such documents are included in the fields searched

Electronic data base consulted during the international search (name of data base and, where practicable, search terms used)

CNABS, VEN, DWPI, CNTXT, OETXT, USTXT, WOTXT, ENTXT, JPTXT, CNKI, IEEE, 读秀, DUXIU, 百度, BAIDU, 百度学术, BAIDU SCHOLAR, Web of Science: 半乳糖, 神经酰胺, 标志物, gal, cer, d18: 1/16: 0, d18: 1/14: 0, 哮喘, 脂, galcer, ceramide, asthma, biomarker, galacto+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | US 2017148618 A1 (UVIC INDUSTRY PARTNERSHIPS INC.) 25 May 2017 (2017-05-25) entire document | 1-10 |
| A | CN 103502820 A (ZORA BIOSCIENCES OY) 08 January 2014 (2014-01-08) entire document | 1-10 |
| A | CN 105044343 A (SHANDONG RESEARCH INSTITUTE OF TUMOUR PREVENTION TREATMENT) 11 November 2015 (2015-11-11) entire document | 1-10 |
| A | CN 105044361 A (SHANDONG RESEARCH INSTITUTE OF TUMOUR PREVENTION TREATMENT) 11 November 2015 (2015-11-11) entire document | 1-10 |
| A | CN 106680473 A (BEIJING CHAO-YANG HOSPITAL, CAPITAL MEDICAL UNIVERSITY) 17 May 2017 (2017-05-17) entire document | 1-10 |
| A | CN 108931587 A (HUZHOU CENTRAL HOSPITAL) 04 December 2018 (2018-12-04) entire document | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **21 March 2024** | **16 April 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/075478**

### C.    DOCUMENTS CONSIDERED TO BE RELEVANT

| Category* | Citation of document, with indication, where appropriate, of the relevant passages | Relevant to claim No. |
|---|---|---|
| A | CN 109212101 A (THE AFFILIATED HOSPITAL OF QINGDAO UNIVERSITY) 15 January 2019 (2019-01-15)<br>    entire document | 1-10 |
| A | CN 113447601 A (BAO FENG BIOTECH (BEIJING) CO., LTD.) 28 September 2021 (2021-09-28)<br>    entire document | 1-10 |

Form PCT/ISA/210 (second sheet) (July 2022)

## INTERNATIONAL SEARCH REPORT
### Information on patent family members

International application No.

**PCT/CN2024/075478**

| Patent document cited in search report | | | Publication date (day/month/year) | Patent family member(s) | | | Publication date (day/month/year) |
|---|---|---|---|---|---|---|---|
| US | 2017148618 | A1 | 25 May 2017 | US | 10020178 | B2 | 10 July 2018 |
| | | | | WO | 2015196303 | A1 | 30 December 2015 |
| CN | 103502820 | A | 08 January 2014 | JP | 2014513286 | A | 29 May 2014 |
| | | | | JP | 5980900 | B2 | 31 August 2016 |
| | | | | CA | 2868372 | A1 | 11 October 2012 |
| | | | | EP | 2694981 | A1 | 12 February 2014 |
| | | | | WO | 2012136272 | A1 | 11 October 2012 |
| | | | | US | 2014031332 | A1 | 30 January 2014 |
| | | | | US | 9664698 | B2 | 30 May 2017 |
| | | | | CN | 103502820 | B | 07 September 2016 |
| | | | | JP | 2016191718 | A | 10 November 2016 |
| CN | 105044343 | A | 11 November 2015 | None | | | |
| CN | 105044361 | A | 11 November 2015 | None | | | |
| CN | 106680473 | A | 17 May 2017 | None | | | |
| CN | 108931587 | A | 04 December 2018 | None | | | |
| CN | 109212101 | A | 15 January 2019 | None | | | |
| CN | 113447601 | A | 28 September 2021 | None | | | |

Form PCT/ISA/210 (patent family annex) (July 2022)

**REFERENCES CITED IN THE DESCRIPTION**

**Patent documents cited in the description**

- CN 2024075478 W
- CN 202311394888